# EUROPEAN PATENT SPECIFICATION

(11) **EP 2 780 080 B1**
(45) Date of publication and mention of the grant of the patent: **13.12.2017**
(21) Application number: 12849633.8
(22) Date of filing: 14.11.2012
(51) Int. Cl.: A61N 1/40

(54) **SYSTEMS FOR SUBCUTANEOUS TREATMENTS**
SYSTEME FÜR SUBKUTANE BEHANDLUNGEN
SYSTÈMES POUR DES TRAITEMENTS SOUS-CUTANÉS

(30) Priority: 16.11.2011 US 201113297608; 16.11.2011 US 201113297934
(43) Date of publication of application: 24.09.2014
(73) Proprietor: BTL Holdings Limited, Limassol PC 3030 (CY)
(72) Inventor: ZARSKY, Jan, Framington, Massachusetts 01702 (US); SCHWARZ, Tomas, 6 Prague (CZ)
(74) Representative: Viering, Jentschura & Partner mbB Patent- und Rechtsanwälte
(86) International application number: PCT/US2012/064942
(87) International publication number: WO 2013/074576

(56) References cited:
- EP-A1- 1 158 919
- US-A- 4 674 481
- US-A- 5 295 955
- US-A- 5 507 790
- US-A- 6 047 215
- US-A1- 2007 106 349
- US-A1- 2009 125 013
- US-A1- 2010 049 261
- US-A1- 2010 100 092
- US-B1- 6 208 903
- US-B1- 6 334 074

## Description

### Field of the Invention

The field of the invention is non-invasive, non-traumatic focused remodeling and downsizing subcutaneous lipid-rich cells, body contouring and skin tightening.

### Background of the Invention

Human skin is composed of three basic elements: the epidermis, the dermis and the hypodermis or so called subcutis. The dermis consists of collagen, elastic tissue and reticular fibers. The hypodermis is the lowest layer of skin and contains hair follicle roots, lymphatic vessels, collagen tissue, nerves and also subcutaneous fat forming an adipose fat tissue. Adipose fat tissue is formed by aggregation of fat cells containing stored lipid (fat). Most fat tissue accumulations result from lipids (fat) primarily from food, when energy intake derived from food exceeds daily energy needs. This may result in an increase in fat cell size or fat cell number or both. Mature fat cells are very large, ranging up to 120 microns in diameter and containing as much as 95% lipid (fat) by volume. The subcutaneous adipose tissue layer may be thin (about 1 cm or less) or in humans of slight or moderate body type.

Excess adipose tissue may be perceived as aesthetically undesirable. Dieting and exercise may result in reduction of adipose tissue and weight loss. However, for most people, the reduction in adipose tissue volume occurs rather unpredictably from all anatomical areas. This can leave the areas intended for reduction, for example, the abdomen, largely unaffected, even after significant body weight loss. Various invasive and non-invasive methods have been developed to remove unwanted subcutaneous fat from specific areas of the body.

Invasive methods, such as liposuction and lipodissolve, can be painful and traumatic, with many undesirable side effects and risks. The non-invasive methods concentrate on the acceleration of the lipolysis as the natural process of the fat reduction. This can be achieved in several ways. One of them is application of pharmaceuticals accelerating the lipolysis. However, when applied topically they tend only to affect the outermost layers of the skin, rarely penetrating to the subdermal vascular plexus. Another method uses radio frequency or ultrasound energy focused on adipose tissue to cause cell destruction and death. These methods tend to damage the melanocyte in the epidermis. The hyperthermic temperatures destroy the target tissues and leave the body to remove the dead cellular and other debris. Non-invasive heating techniques have also been used. These involve heating the adipose fat tissue to about 40°C or more via direct contact with a heating element. These non-invasive methods have certain disadvantages as well, and have been used with varying degrees of success.

The Patent Application EP-A1-1158919 discloses a system for treating subcutaneous adipose tissue of a patient comprising capacitive electrodes to be applied on the skin.

Accordingly, there is need for improved systems for subcutaneous treatments.

### BRIEF STATEMENT OF THE INVENTION

The invention is a system as defined in the appended claims and can be used in a method for treating subcutaneous tissue as describing in the following paragraphs. A method for treating subcutaneous tissue includes positioning one or more applicators adjacent to the skin of a patient, but not touching the skin. Electromagnetic energy is transmitted from the applicators into the subcutaneous tissue. The subcutaneous tissue is heated via the electromagnetic energy. The subcutaneous tissue may be remodeled. The volume of lipid-rich cells in the subcutaneous tissue may be reduced via the heating. The electromagnetic waves may be applied in a pulsed mode or in a continuous mode. The skin may optionally be actively cooled, without contacting the skin. This method may also be used for tightening the skin and for remodeling collagen tissue in the subcutaneous tissue. With the applicator not touching the skin, the need for cooling the skin, and bio-compatibility factors are avoided.

Another method for treating subcutaneous tissue includes using one or more applicators without continuously moving the applicator. One or more applicators may be supported on fixtures or holders, rather than hand-held. Exclusive and continuous attention to the treatment by an experienced user of the system may not necessarily be required.

### BRIEF DESCRIPTION OF THE FIGURES

Fig. 1 is a schematic diagram of a system for controlled deep heating of sub dermal tissues.
Fig. 2 is a schematic view of a trans-regional course of electromagnetic field;
Figs. 3 and 4 are schematic examples of positioning of electrodes shown in Fig. 1.

### DETAILED DESCRIPTION

Prior art methods generally require direct contact of an applicator onto the skin. This in turn typically also requires use of active skin cooling elements. Direct skin contact can also raise bio-compatibility issues with the applicator material and further requires high sanitary standards, since the applicators are used for treatment of different patients. The practitioner must also be skilled in using the applicators since there is a risk of burning the patient. With the prior art methods, the practitioner must also continuously move the applicator, to reduce the risk of burning the patient.

These disadvantages are overcome by transmitting electromagnetic energy into the sub subcutaneous tissue, without physical contact with the patient. Treatment methods that avoid contact between the applicator and the skin enable simultaneous treatment of large areas of human body. It also avoids the need for artificial cooling of the skin. In the present non-contact methods, the skin may be sufficiently cooled passively by ambient air. Optionally, the skin may be cooled via a stream of chilled or room temperature air. The present methods also do not require use of cooling fluids and gels. This reduces costs and increases patient comfort.

In one aspect, the present methods work on the principle of selective deep heating of the human tissue containing low volume of water, such as adipose tissue. Radiant energy may be provided to the sub dermal tissue by one or more capacitive electrodes generating an electromagnetic field. Selective heating in the dermis occurs due to dielectric losses.

In a continuous application mode, the electromagnetic field is applied continuously, which provides a maximum amount of heating. Using a pulse mode, the heat is local and typically limited to about 400 W. With the pulse mode, a high frequency field is applied in short intervals (typically (50-2000 µs) and on various pulse frequencies (typically 50 to 1500 Hz). The maximum output with the continuous method is typically limited to 200 W.

The increase of the temperature in the dermal and the sub dermal tissues also affects the triple-helix structure of collagen fibers contained in such tissues. This may result in remodeling and rejuvenation of collagen, increase of skin density and dermal thickening based on neocollagenesis. Skin tightening may also be achieved.

Remodeling and reducing the volume of subcutaneous lipid-rich cells, and skin tightening in the targeted areas, can change the overall appearance of the body, for use in body contouring and body reshaping.

Electromagnetic energy is provided through the skin to the underlying sub dermal tissue, without contacting the skin. The radiant energy is converted into heat in the sub dermal tissue. The radiant energy enables focused heating of the subcutaneous adipose tissue and sub dermal collagen tissue, leading to accelerating lipolysis. At the same time the triple-helix structure of collagen fibers may result in remodeling and/or rejuvenation of collagen, increase of skin density and dermal thickening based on neocollagenesis. Subcutaneous lipid-rich cells may be remodeled and/or reduced in volume, contouring and tightening skin tissue.

Another method allows for treatment without a need for continuous movement of the applicator. The step and repeat movements of the applicator over a grid pattern on the patient's skin is obviated. With the applicator applying heating over a larger area, constant movement of the applicator is not needed. The applicator may remain in a stationery position relative to the patient for several seconds or longer, for example, for at least 5, 10, 30, 60, 120 or 240 seconds, or longer. Non-contacting methods also enable simultaneous treatment of large areas of human body. It also avoids the need for artificial cooling of the skin. In the present methods where the applicator does not contact the skin, the skin may be sufficiently cooled passively by circulating air. Optionally, the skin may be cooled via a stream of chilled or room temperature air. The present methods also do not require use of cooling fluids and gels. This reduces costs and increases patient comfort.

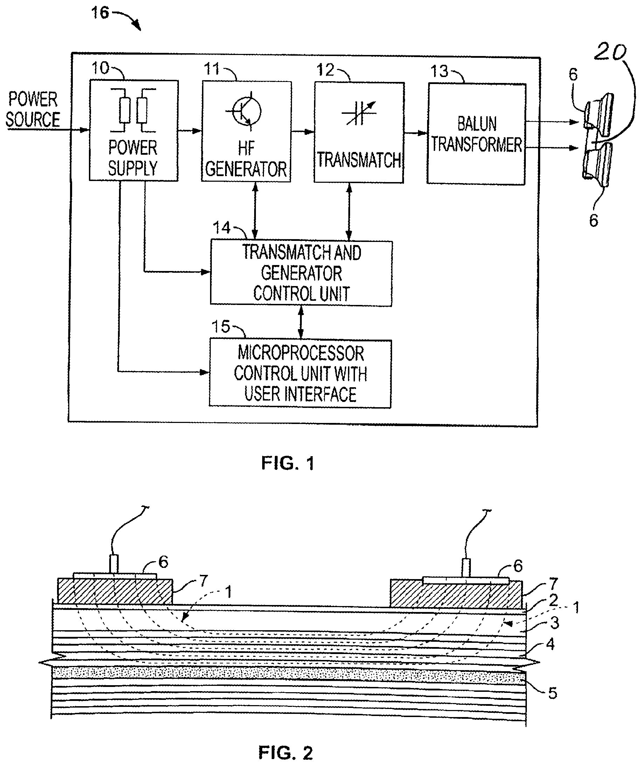

Referring now to Fig. 1, a system 16 applies electromagnetic energy through a skin layer, such as the epidermis, and to the underlying sub dermal tissue, and underlying collagen tissue, causing acceleration of lipolysis and collagen remodeling. The system may include 6 blocks. The power supply 10 is connected to a power source. An HF generator (high frequency generator) 11 and a transmatch and generator control unit 14, and a microprocessor control unit with user interface 15, are connected to the power supply 10. The HF generator 11 may generate an electromagnetic field at 13.56 or 40.68 or 27.12 MHz, or 2.45 GHz or optionally at other frequencies as well. The 13.56, 27.12 and 40.68 MHz and 2.45 GHz frequencies avoid creating radio interference, as these frequencies are exclusively assigned as free or open frequencies.

The microprocessor control unit with user interface 15 provides communication between the transmatch and generator control unit 14 and user interface, which may be a touch screen on the device display.

The transmatch and generator control unit 14 receives information from the operator via the control unit and regulates the operation of the HF generator 11 and the transmatch 12. The transmatch transmits HF to a balun transformer 13, which converts unbalanced impedance to balanced impedance. This processed signal goes to two capacitive applicators 6, which may be positioned approximately 2-3 cm above the surface of the skin.

Fig. 2 is a schematic representation of a heat distribution under the skin. One or more applicators 6 create an electromagnetic field. This electromagnetic field crosses the air gap 25 between the applicator and the patient's skin, and penetrates through the skin 2, subcutaneous fat 3 and muscle 4 or the bone 5. Capacitive applicators 6 provide deep heating, which heats selectively only structures with low volume of water. A spacer 7 such as a towel, gauze pad, foam pad, cloth pad, etc. may be placed on the skin, with the applicator then placed on top of the spacer 7. This automatically sets the separation distance between the applicator and the skin, and prevents the applicator from touching the skin.

If used, the spacer 7 may be made of various dielectric or electrically nonconductive materials. The spacer 7 is typically dry in use. Alternatively, a reusable or a disposable spacer may be attached to the applicator. For example, the spacer may comprise posts, a frame, or other structure on the applicator that contacts the skin, while keeping the active surface of the applicator spaced apart from the skin. As described and claimed here, such spacing elements are additional elements and not part of applicator. The methods may be performed with no part or surface of the actuator, including any attachment on the applicator, in contact with the skin.

A selective heating process is observed in the dermis 3 due to dielectric losses. Dielectric loss is created as part of an AC electromagnetic field power is converted to heat in the dielectric. During this process, polar molecules rotate, and their movement produces the thermal energy. Skin and muscle are largely not affected by electromagnetic field 1 as they contain water and the blood circulation provides for cooling. Bone 5 gets little if any heating because the applicators 6 are positioned to create a field only on the upper structures. The lipid cells of the adipose tissue contain less water than the surrounding tissue and are therefore heated at higher level than the surrounding tissue.

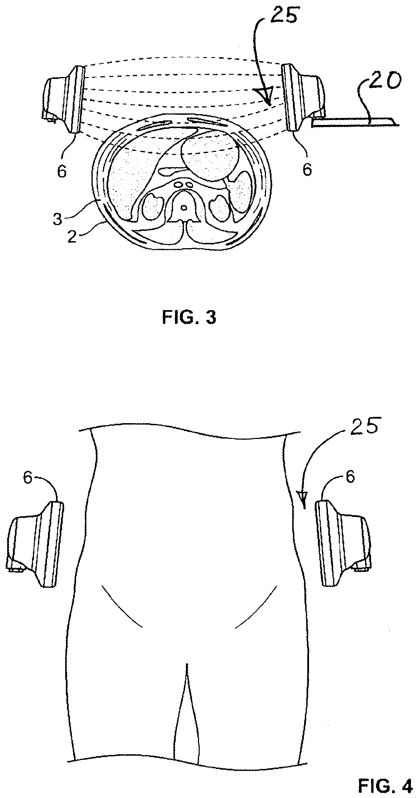

Figs. 3 and 4 are schematic examples of positioning of the applicators or electrodes 6 providing radiant energy through the skin 2 to subcutaneous fat 3. The electrodes are positioned approximately 2-3 cm above the surface of the skin or placed onto a spacer 7 which is in contact with the skin surface, as shown in Fig. 5. The spacer 7, if used, may correspondingly typically be about 0.5 to1 cm thick. The applicator 6 may be temporarily fixed in position relative to the patient, if desired, for example on a mechanical fixture or holder.

It is not necessary in each instance for the applicator to be continuously moving during the procedure. This makes the procedure easier to perform, since user need not constantly keep moving the applicator over the patient's skin. Consequently, the user can accordingly simultaneously attend to other needs of a patient. The applicator 6 may have a relatively large surface area, so that the field 1 is distributed more widely through the subcutaneous tissue. For example, the applicator may have a surface area of at least about 10, 15, 30, 50, 100, or 150 cm².

If more than one applicator is used, applicators may be positioned on opposite sides of the patient. A spacer may be positioned between one or more applicator and the skin of the patient.

Methods may include one or more of the following steps: positioning a spacer or an air gap in between the applicator and the skin of the patient; transmitting electromagnetic energy in the range of 13.553 - 13.567 or 26.957 - 27.83 or 40.66 - 40.70 MHz or 2.4 - 2.5 GHz from the applicator into the subcutaneous tissue; and placing or holding the applicator in a fixed position relative to the tissue for at least 10 seconds; optionally with the applicator not touching the skin of the patient. If two or more applicators are used, the applicators may be positioned on opposite sides of the patient. The applicator may be two or more capacitive electrodes.

The electromagnetic waves may be applied in a continuous mode, or pulse mode with a power range of e.g. 30 - 400 W per pulse.

## Claims

1. A noncontact cosmetic treatment system (16) for treating subcutaneous tissue of a patient having a volume of lipid-rich cells, comprising:
a power supply (10), a HF generator (11), a transmatch (12), a balun transformer (13), a transmatch and generator control unit (14), a microprocessor control unit with a user interface (15), and an applicator (6);
wherein the transmatch (12) is configured to process a signal from the HF generator (11) and transmit the processed HF signal to the balun transformer (13),
wherein the balun transformer (13) is configured to convert unbalanced impedance to balanced impedance and then this processed signal goes to the applicator (6);
wherein the applicator (6) comprises at least one pair of two balanced capacitive electrodes;
wherein the microprocessor control unit with the user interface (15) are configured to provide communication to the transmatch and generator control unit (14);
wherein the transmatch and generator control unit (14) is configured to regulate the operation of the HF generator (11) and the transmatch (12);
wherein the applicator (6) has a surface area of at least 50 cm²;
wherein the applicator (6) is configured to be positioned spaced apart from the skin (2) of the patient by an air gap;
wherein the applicator (6) comprises a mechanical holder configured to temporarily fix the applicator (6) in position relative to the patient;
and wherein the applicator (6) is configured to transmit the corresponding HF waves into the subcutaneous tissue to thereby heat the subcutaneous tissue via the HF waves.

2. The system (16) of claim 1, wherein the skin of the patient is cooled by stream of chilled or room temperature air.

3. The system (16) of claim 1, wherein the skin of the patient is cooled via air circulating through the air gap and the temperature of skin is increased to 32-45°C.

4. The system (16) one of claims 1-3, further configured to apply the HF waves with pulse width between 50-2000 micro seconds and pulse frequency range from 50-1500 Hz.

5. The system (16) of one of claims 1-4, further configured to apply the HF waves in a pulsed mode with a power range of 30-400 W per pulse.

6. The system (16) of one of claims 1-4, further configured to apply the HF waves in a continuous mode.

7. The system (16) of one of claims 1-6, further configured to transmit HF waves in the range of 13.553-13.567 or 26,957-27,283 or 40.66-40.70 MHz or 2.4-2.5 GHz from the applicator (6) into the subcutaneous tissue.

8. The system (16) of one of claim 1-7, further comprising a spacer pad (7) having a frame, to be positioned between the applicator/s (6) and the skin of the patient.

9. The system (16) of claim 8 with the spacer pad (7) comprising dielectric cloth or foam material.

10. The system (16) of one of claims 1-9 with the applicator (6) having a surface area of at least 100 cm².

11. The system (16) of one of claims 1-10 with the applicator (6) having a surface area of at least 150 cm².

## Patentansprüche

1. Ein berührungsloses kosmetisches Behandlungssystem (16) zur Behandlung von subkutanem Gewebe eines Patienten, das ein Volumen an lipidreichen Zellen hat, aufweisend:
eine Stromversorgung (10), einen HF-Generator (11), einen Transmatch (12), einen Balun-Transformator (13), eine Transmatch-und-Generator-Steuereinheit (14), eine Mikroprozessor-Steuereinheit mit einer Benutzerschnittstelle (15) und einen Applikator (6),
wobei der Transmatch (12) eingerichtet ist zum Verarbeiten eines Signals von dem HF-Generator (11) und zum Übertragen des verarbeiteten HF-Signals an den Balun-Transformator (13),
wobei der Balun-Transformator (13) eingerichtet ist, um eine asymmetrische Impedanz in eine symmetrische Impedanz umzuwandeln, und dieses verarbeitete Signal wird dann an den Applikator (6) übertragen,
wobei der Applikator (6) mindestens ein Paar von zwei symmetrischen kapazitiven Elektroden aufweist,
wobei die Mikroprozessor-Steuereinheit mit der Benutzer-Schnittstelle (15) eingerichtet ist zum Bereitstellen einer Kommunikation zu der Transmatch-und-Generator-Steuereinheit (14);
wobei die Transmatch-und-Generator-Steuereinheit (14) eingerichtet ist zum Regulieren des Betriebs des HF-Generators (11) und des Transmatch (12),
wobei der Applikator (6) einen Flächenbereich von mindestens 50 cm² hat,
wobei der Applikator (6) eingerichtet ist, um durch einen Luftspalt im Abstand von der Haut (2) des Patienten angeordnet zu sein,
wobei der Applikator (6) eine mechanische Halterung aufweist, die eingerichtet ist, um den Applikator (6) temporär in einer Position relativ zu dem Patienten zu fixieren,
und wobei der Applikator (6) eingerichtet ist, um die entsprechenden HF-Wellen in das subkutane Gewebe zu übertragen, um dadurch das subkutane Gewebe mittels der HF-Wellen zu erwärmen.

2. System (16) gemäß Anspruch 1, wobei die Haut des Patienten gekühlt wird von einem Strahl gekühlter Luft oder Luft mit Raumtemperatur.

3. System (16) gemäß Anspruch 1, wobei die Haut des Patienten mittels einer Luftzirkulation durch den Luftspalt gekühlt wird und die Hauttemperatur auf 32 °C bis 45 °C erhöht wird.

4. System (16) gemäß einem der Ansprüche 1 bis 3, ferner eingerichtet zum Applizieren der HF-Wellen mit einer Pulsweite zwischen 50 bis 2000 Mikrosekunden und einem Puls-Frequenzbereich von 50 bis 1500 Hz.

5. System (16) gemäß einem der Ansprüche 1 bis 4, ferner eingerichtet zum Applizieren der HF-Wellen in einem Pulsbetrieb mit einem Leistungsbereich von 30 bis 400 W pro Puls.

6. System (16) gemäß einem der Ansprüche 1 bis 4, ferner eingerichtet zum Applizieren der HF-Wellen in einem kontinuierlichen Betrieb.

7. System (16) gemäß einem der Ansprüche 1 bis 6, ferner eingerichtet zum Übertragen von HF-Wellen in dem Bereich von 13,553 bis 13,567 oder 26,957 bis 27,283 oder 40,66 bis 40,70 MHz oder 2,4 bis 2,5 GHz von dem Applikator (6) in das subkutane Gewebe.

8. System (16) gemäß einem der Ansprüche 1 bis 7, ferner aufweisend ein Abstands-Pad (7) mit einem Rahmen zum Angeordnet-werden zwischen dem/den Applikator(en) (6) und der Haut des Patienten.

9. System (16) gemäß Anspruch 8, wobei das Abstands-Pad (7) ein dielektrisches Stoff- oder Schaummaterial aufweist.

10. System (16) gemäß einem der Ansprüche 1 bis 9, wobei der Applikator (6) einen Flächenbereich von mindestens 100 cm² hat.

11. System (16) gemäß einem der Ansprüche 1 bis 10, wobei der Applikator (6) einen Flächenbereich von mindestens 150 cm² hat.

## Revendications

1. Système de traitement cosmétique sans contact (16) pour le traitement d'un tissu sous-cutané d'un patient ayant un volume de cellules riches en lipides, comprenant :
une alimentation (10), un générateur HF (11), un transmatch (12), un transformateur balun (13), une unité de commande de transmatch et de générateur (14), une unité de commande de microprocesseur avec une interface utilisateur (15) et un applicateur (6) ;
dans lequel le transmatch (12) étant conçu pour traiter un signal provenant du générateur HF (11) et transmettant le signal HF traité au transformateur balun (13) ;
dans lequel le transformateur balun (13) étant conçu pour convertir une impédance déséquilibrée en une impédance équilibrée puis cette signal traité est transmis à l'applicateur (6) ;
dans lequel l'applicateur (6) comprenant au moins une paire de deux électrodes capacitives équilibrées ;
dans lequel l'unité de commande de microprocesseur avec l'interface utilisateur (15) étant conçue pour établir une communication avec l'unité de commande de transmatch et de générateur (14) ;
dans lequel l'unité de commande de transmatch et de générateur (14) étant conçue pour réguler le fonctionnement du générateur HF (11) et du transmatch (12) ;
dans lequel l'applicateur (6) présentant une superficie d'au moins 50 cm² ;
dans lequel l'applicateur (6) étant conçu pour être éloigné de la peau (2) du patient par une couche d'air ;
dans lequel l'applicateur (6) comprenant un support mécanique conçu pour fixer temporairement l'applicateur (6) en position par rapport au patient ;
et dans lequel l'applicateur (6) étant conçu pour transmettre les ondes HF correspondantes vers le tissu sous-cutané afin de chauffer le tissu sous-cutané via les ondes HF.

2. Système (16) selon la revendication 1, dans lequel la peau du patient est refroidie par un flux d'air refroidi ou à température ambiante.

3. Système (16) selon la revendication 1, dans lequel la peau du patient est refroidie par une circulation d'air à travers la couche d'air et la température de la peau est augmentée de 32 à 45 °C.

4. Système (16) selon l'une des revendications 1 à 3, conçu en outre pour appliquer les ondes HF avec une largeur d'impulsion entre 50 et 2000 micro-secondes et une plage de fréquence d'impulsion de 50 à 1500 Hz.

5. Système (16) selon l'une des revendications 1 à 4, conçu en outre pour appliquer les ondes HF en mode pulsé avec une plage de puissance de 30 à 400 W par impulsion.

6. Système (16) selon l'une des revendications 1 à 4, conçu en outre pour appliquer les ondes HF en mode continu.

7. Système (16) selon l'une des revendications 1 à 6, conçu en outre pour transmettre des ondes HF dans la plage de 13,553 à 13,567 ou de 26,957 à 27,283 ou de 40,66 à 40,70 MHz ou de 2,4 à 2,5 GHz à partir de l'applicateur (6) vers le tissu sous-cutané.

8. Système (16) selon l'une des revendications 1 à 7, comprenant en outre un bloc d'espacement (7) ayant un cadre pour être positionnée entre l'applicateur (6) et la peau du patient.

9. Système (16) selon la revendication 8, le bloc d'espacement (7) comprenant une étoffe diélectrique ou un matériau alvéolaire.

10. Système (16) selon l'une des revendications 1 à 9, l'applicateur (6) présentant une superficie d'au moins 100 cm².

11. Système (16) selon l'une des revendications 1 à 10, l'applicateur (6) présentant une superficie d'au moins 150 cm².
